# FASCICULE DE BREVET EUROPEEN

(11) **EP 2 255 182 B1**
(45) Date de publication et mention de la délivrance du brevet: **12.12.2018**
(21) Numéro de dépôt: 09713368.0
(22) Date de dépôt: 11.02.2009
(51) Int. Cl.: G01N 33/46, G01N 33/14

(54) **PROCÉDÉ DE CONTRÔLE DE CONTAMINATION DES BARRIQUES**
VERFAHREN ZUR ÜBERWACHUNG DER VERUNREINIGUNG VON FÄSSERN
METHOD OF MONITORING THE CONTAMINATION OF BARRELS

(30) Priorité: 22.02.2008 FR 0851143
(43) Date de publication de la demande: 01.12.2010
(73) Titulaire: Laboratoire Excell, 33700 Merignac (FR)
(72) Inventeur: CHATONNET, Pascal, 33000 Bordeaux (FR); BOUTOU, Stéphane, 33420 Jugazan (FR)
(74) Mandataire: Ipside
(86) Numéro de dépôt international: PCT/EP2009/051565
(87) Numéro de publication internationale: WO 2009/103640

(56) Documents cités:
- MOUTOUNET M ET AL: "Analysis by HPLC of Extractable Substances in Oak Wood - Application to a Chardonnay Wine // Analyse CLHP d'extractibles du bois de chêne - application a un vin de Chardonnay" SCIENCES DES ALIMENTS, LAVOISIER ABONNEMENTS, PARIS, FR, vol. 9, no. 1, 1 janvier 1989 (1989-01-01), pages 35-51, XP009064355 ISSN: 0240-8813
- PIZARRO ET AL: "Multiple response optimisation based on desirability functions of a microwave-assisted extraction method for the simultaneous determination of chloroanisoles and chlorophenols in oak barrel sawdust" JOURNAL OF CHROMATOGRAPHY, ELSEVIER SCIENCE PUBLISHERS B.V. AMSTERDAM, NL, vol. 1132, no. 1-2, 3 novembre 2006 (2006-11-03), pages 8-14, XP005704865 ISSN: 0021-9673
- CHATONNET P ET AL: "Prévention et détection des contaminations par Brettanomyces au cours de la vinification et de l'élevage des vins // Prevention and Detection of Brettanomyces Contamination in Wine Making" REVUE FRANCAISE OENOLOGIE, vol. 179, 1 novembre 1999 (1999-11-01), pages 20-24, XP009080570

## Description

La présente invention a trait à un procédé de contrôle de contamination des barriques qui sont destinées de manière générale à l'élevage d'une boisson alcoolisée telle que le vin, l'eau-de-vie, et plus particulièrement au contrôle de certains contaminants présents dans les pièces de bois des barriques, rendant ces derniers impropres à l'utilisation.

Mais il est évident que le procédé de l'invention peut s'appliquer de manière plus générale au contrôle de contamination de tout type de contenant réalisé à base de bois destiné à l'élevage d'une boisson alcoolisée.

On sait que le passage d'un vin, d'un moût, d'une boisson alcoolisée dans un contenant de bois et notamment en barrique permet à celui-ci de se charger petit à petit d'un certain nombre de constituants du bois par des phénomènes de diffusion. Les constituants cédés varient bien évidemment en fonction de l'origine du bois, en fonction du brûlage des douelles.

Le document Moutounet M et al., SCIENCES DES ALIMENTS, vol. 9, no. 1, janvier 1989, pages 35-51, divulgue une étude analytique en CLHP (chromatographie liquide à haute performance) porté sur des macérations alcooliques de copeaux, une solution hydroalcoolique stockée en fût de chêne et un vin de Chardonnay vinifié et élevé en fût de chêne pendant 12 mois, afin de mieux cerner l'influence du bois de chêne sur la composition des vins. Le document Chatonnet P et al., REVUE FRANÇAISE OENOLOGIE, vol. 179, novembre 1999, pages 20-24, concerne la prévention et détection des contaminations par Brettanomyces (et leurs métabolites spécifiques tels que les éthyl-phénols) au cours de la vinification et de l'élevage des vins.

La qualité du vin est donc intimement liée à la qualité des bois qui seront utilisés pour fabriquer la barrique neuve. Les bois contaminés peuvent donc également céder à la boisson au cours de l'élevage des contaminants tels que des composés de la famille des phénols, des composés de la famille des chloroanisoles, provoquant ainsi une altération organoleptique et/ou même un risque pour la santé de l'homme.

Dans le cas des barriques neuves, cette contamination peut avoir lieu au cours du séchage à l'air libre des merrains qui sont stockés dans un parc à bois et qui peut durer 1 à 3 ans. Elle peut être également due aux composés utilisés dans le traitement des bois.

Pour remédier à ces problèmes de contamination, avant la fabrication des barriques, les tonneliers procèdent à un contrôle de contamination qui consiste à prélever localement des morceaux de bois et de manière aléatoire parmi les merrains stockés ou les douelles composant la barrique finie pour savoir si les bois sont contaminés avant de procéder à la fabrication des barriques.

Toutefois on sait que la contamination n'affecte pas de manière uniforme les pièces de bois, parfois seule une petite zone d'une pièce de bois peut être affectée par une contamination extrêmement localisée et plus ou mois profonde. En outre, seule une fraction des contaminants présents dans le bois est susceptible de migrer dans le vin. En outre, les prélèvements effectués sur la barrique finie sont susceptibles d'affecter son intégrité.

Un tel procédé de contrôle de contamination de l'état de la technique ne permet donc pas de déterminer de manière exacte et quantitative le degré de contamination. En outre, les mesures obtenues qui reflètent la contamination d'une zone localisée ne sont pas représentatives de la totalité de la surface de bois qui sera mise en contact avec le vin.

En fonction des besoins et des goûts, les viticulteurs sont amenés à changer leurs barriques tous les deux ou trois ans, voir tous les ans. Ces barriques usagées sont généralement réutilisées pour la vinification de certain type de vin dont l'exigence des qualités organoleptiques est moindre. Lorsque la barrique saine a été utilisée pour élever un vin contaminé, les composés contenus dans ce vin contaminé pourront également contaminer la barriques en étant retenus dans les pores du bois. Cette barrique pourra alors à son tour contaminer un vin sain lors de son élevage.

Le procédé de contrôle de contamination tel que décrit ci-dessus n'est pas adapté pour déterminer le degré de contamination des barriques usagées. Il nécessite des prélèvements de bois sur les barriques usagées, ce qui entraîne soit un démontage de la barrique usagée, voir même la destruction de la barrique.

Aussi la présente invention vise à fournir un procédé qui permet de déterminer la contamination de la totalité ou la quasi-totalité de la surface des barriques qui sera amenée en contact direct avec le vin à élever, contrairement aux mesures du procédé existant qui sont des mesures aléatoires et non représentatives de la surface réellement en contact avec le produit à élaborer. Le procédé proposé est rapide et simple à mettre en oeuvre, en outre il ne nécessite pas le démontage des barriques.

Le concept principal de la présente invention est de pouvoir déterminer la contamination directement sur des barriques neuves finies prêtes pour l'élevage ou des barriques usagées en partant de l'idée que certains contaminants tels que les chloroanisoles et les chlorophénols par exemple sont facilement extractibles, c'est-à-dire susceptible de migrer facilement et rapidement dans les vins contenus dans les barriques.

Aussi, au lieu de prélever des échantillons de bois de manière aléatoires, la solution de la présente invention permet d'analyser directement les eaux d'échaudage utilisées lors de l'étape de contrôle d'étanchéité des barriques ou de préparation au remplissage par les utilisateurs finaux. Cette solution a l'avantage de prendre en compte facilement la totalité de la superficie d'échange de la coque interne, d'être rapide car elle ne nécessite pas d'opérations supplémentaire de la part des tonneliers. En outre, le fait d'utiliser des eaux d'échaudage permet d'extraire plus facilement à chaud les contaminants tout en étant non destructive.

A cet effet, l'invention a pour objet un procédé de contrôle de contamination d'un lot comprenant au moins un contenant en bois (1), ledit contenant étant destiné à l'élevage d'une boisson alcoolisée.

### Selon l'invention, ledit procédé comporte

Une phase de préparation d'échantillon destiné au dosage des contaminants dans laquelle :
a) on récupère un échantillon d'un volume d'eau qui a été mis en contact sous agitation avec toute la surface interne dudit au moins un contenant du lot à contrôler, ledit volume d'eau étant utilisé une seule fois pour chaque contenant ;
b) ledit échantillon récupéré dans le cas où le lot comporte un seul contenant ou l'assemblage à proportion égale de l'ensemble des échantillons d'eaux récupérés pour chaque contenant du lot à contrôler est ensuite envoyé dans un récipient (3) fermé pour une phase de dosage des contaminants ; et une phase de dosage des contaminants dans laquelle :
c) on détermine au préalable une valeur seuil de la concentration des contaminants correspondant à un état de risque de contamination du contenant ;
d) on réalise un dosage des contaminants de l'échantillon récupéré ou de l'assemblage obtenu;
e) on réalise une étude comparative entre la concentration des contaminants mesurée par rapport à la valeur seuil pour déterminer si le lot est contaminé; et, avantageusement,
f) lorsque la concentration est supérieure à la valeur seuil correspondant à un état contaminé du lot et lorsque ledit lot comprend au moins deux contenants, on répète les opérations des étapes a) à e) pour chacun des contenants dudit lot de manière à localiser le ou les contenant(s) contaminés du lot.

Ainsi comme on vient de le voir, l'idée de base de la présente invention est de déterminer le risque de contamination à l'utilisation à partir du dosage des contaminants présents dans un échantillon d'eaux qui a été mise en contact avec toute la surface interne de chaque contenant du lot.

La concentration des contaminants dans les eaux permet de remonter au degré de contamination des contenants.

La solution proposée dans la présente invention peut être appliquée pour contrôler un lot comprenant un ou plusieurs contenant(s).

Afin de réduire le coût du procédé, il est possible de ne pas appliquer le procédé pour chaque contenant individuellement mais pour le lot entier. On procèdera donc à récupérer les eaux de tous les contenants du lot et on mélange les eaux de manière à effectuer un dosage des contaminants pour chaque lot.

Le nombre de contenants par lot dépend du degré de risque de contamination que l'on souhaite prendre en compte pour effectuer le dosage. En effet, plus ce nombre est important, plus le mélange sera dilué. En raison des limites de détection et de quantification qui dépend de la technique d'analyse utilisée, et en fonction du type de contaminant, le nombre de contenants est compris entre 5 et 20 ou plus pour chaque lot.

A titre d'exemple, pour le dosage du risque de contamination de 2,4,6-trichloroanisoles (TCA) dont le seuil de défaut est autour de 3 ng/L, il est déconseillé d'assembler au-delà de 20 contenants. Et pour une sécurité accrue, un assemblage de 5 contenants est souhaitable.

On entend par seuil de défaut, le seuil correspondant à une concentration de contaminant dans le vin au de-là de laquelle le défaut organoleptique est perceptible.

De préférence, ledit procédé comporte une étape b') dans laquelle on remplit le récipient avec ledit échantillon récupéré ou ledit assemblage de l'ensemble, ledit récipient étant fermé de manière étanche au moyen d'un bouchon inerte, ledit récipient étant ensuite envoyé pour la phase de dosage des contaminants.

Dans une forme avantageuse de l'invention, on incorpore un agent stabilisant dans le récipient avant son bouchage. Cet agent stabilisant est un antiseptique minéral qui peut être du fluorure de sodium, du métabisulfite de sodium, silicate de sodium ou du benzoate de sodium. La concentration de cet agent stabilisant est de préférence inférieure à 100 mg/l.

Même si le récipient est fermé hermétiquement et stabilisé, il est conseillé d'envoyer le récipient contenant le mélange à analyser dans les 24 heures suivant le prélèvement du mélange d'eaux chaudes ou à température ambiante afin d'éviter la dégradation ou la contamination de l'échantillon par le milieu environnant durant son stockage et son transport avant l'analyse.

De préférence, le temps de la mise en contact de l'eau avec la surface interne du contenant est au moins supérieur à 100 secondes pour extraire les molécules piégées dans les pores du bois du contenant sous agitation constante pour bien prendre en compte toute la surface du contenant en bois.

De préférence, l'eau utilisée dans l'étape a) est de l'eau portée à une température contrôlée comprise entre 50 et 80 °C ou de l'eau à température ambiante.

De préférence, on incorpore de l'éthanol dans l'eau de manière à ajuster la solvatation de l'eau.

Avantageusement la qualité de l'eau utilisée lors de l'étape a) est contrôlée périodiquement, au moins une fois par mois, permettant ainsi d'assurer que l'éventuelle contamination provient effectivement du bois contaminé et non de l'eau.

Dans une forme particulièrement avantageuse, l'eau récupérée lors de l'étape a) est de l'eau utilisée pour contrôler l'étanchéité du contenant. Ainsi pour les tonneliers fabricants, la solution de la présente invention n'implique pas d'étape supplémentaire, puisque l'étape de contrôle d'étanchéité est une étape obligatoire dans le processus de la fabrication d'un contenant en bois tel qu'une barrique. En outre la phase de préparation de l'échantillon pour le dosage ne nécessite pas de compétence particulière de la part de l'opérateur.

De préférence, les contaminants à doser sont constitués par l'un des constituants du groupe des haloanisoles comprenant le 2,4,6-trichloroanisole (TCA), le 2,4,6-tribromoanisole (TBA), le 2,3,4,6-tétrachloroanisole (TeCA), et le pentachloroanisole (PCA), l'un des constituants du groupe des chlorophénols comprenant le 2,4,6-trichlorophénol (TCP), le 2,3,4,6 tétrachlorophénol (TeCP), et le pentachlorophénol (PCP), de la famille des phénols volatils comprenant le 4-éthylphénol et le 4-éthylguaïacol, l'un des constituants du groupe d'hydrocarbure aromatique polycyclique, l'acide acétique ou l'acétate d'éthyle.

Dans une autre forme particulièrement avantageuse de la présente invention, le procédé comprend une étape supplémentaire dans laquelle pour chaque contaminant, on réalise une courbe de corrélation entre la concentration du contaminant dans la boisson après avoir passé un temps déterminé dans le contenant et la concentration du contaminant correspondant du contenant, de manière à extraire une valeur seuil de rejet au contrôle de contamination correspondant à une teneur en contaminant susceptible d'affecter la qualité organoleptique de la boisson.

Cette valeur seuil de rejet au contrôle de contamination pour le contaminant TCA est comprise entre 0.2 ng/L et 1.0 ng/L.

On entend donc ici par concentration du contaminant dans la boisson, la concentration du contaminant effectivement migré dans la boisson en contact avec la surface des bois pendant un temps déterminé. La durée peut être de 6 mois à deux ans.

On entend par concentration du contaminant du contenant, la concentration du contaminant déterminé à partir des échantillons d'eaux mis en contact avec la surface interne du contenant.

De manière générale, le présent procédé peut être appliqué à tout type de contenants en bois, il est particulièrement adapté pour les barriques en chêne neuves ou usagées destinées à l'élevage des vins rouges.

D'autres avantages et caractéristiques de l'invention apparaîtront avec la description de certains de ses modes de réalisations particuliers, cette description étant effectuée à titre non limitatif à l'aide des figures sur lesquelles :
- la figure 1 montre un organigramme des différentes étapes du procédé de contrôle de contamination selon l'invention ;
- la figure 2 montre des résultats de contrôle de contamination illustrant le procédé pour 6 lots de barriques, chaque lot étant constitué de 5 barriques ;
- la figure 3 montre des résultats de contrôle de contamination pour chaque barrique individuellement d'un lot contaminé,
- la figure 4 montre une courbe de corrélation entre la concentration de TCA migré effectivement dans le vin et la concentration de TCA contrôlée de la barrique.

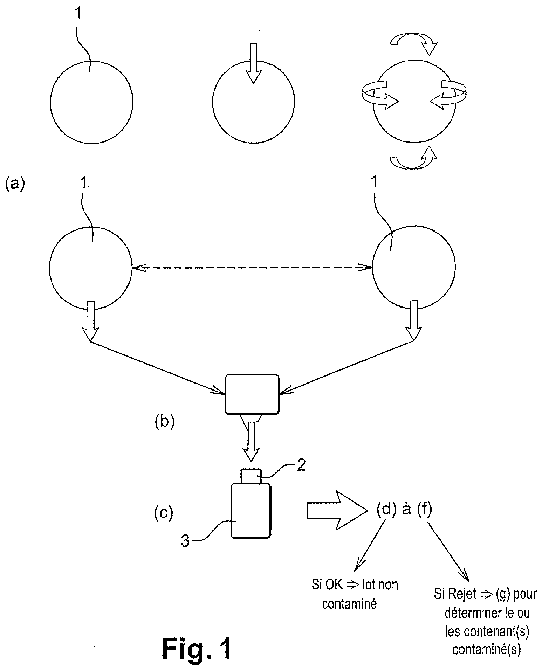

La figure1 illustre les différentes étapes d'un exemple de réalisation du procédé de la présente invention telle que décrite ci-dessus.

Dans une première étape (a), on récupère un échantillon d'un volume d'eau chaude qui a été mise en contact sous agitation avec toute la surface interne de chaque barrique 1 d'un lot à contrôler. Ce volume d'eau chaude est utilisé qu'une seule fois pour chaque barrique et est portée à une température contrôlée. Cette eau chaude a été envoyée sur la surface interne de la barrique avec une pression contrôlée comprise entre 0,2 et 0,5 bars. De préférence, l'eau chaude utilisée pour cette étape est de l'eau pure chaude portée à une température comprise entre 50 et 80 °C.

Nous pouvons envisager également l'utilisation de l'eau à température ambiante. Toutefois l'efficacité de l'extraction des contaminants est réduite.

Dans une forme avantageuse de l'invention, il est possible d'incorporer de l'éthanol dans l'eau pure jusqu'à 10 à 13 % permettant d'accroître par ses propriétés de solvatation, l'extraction des contaminants ciblés et d'abaisser le seuil de détection analytique. Le pourcentage de l'éthanol dans l'eau peut être ajusté de manière quelconque en fonction du procédé de contrôle et du seuil de détection analytique.

L'étape (a) est effectuée pour chacun des contenants du lot à contrôler.

Dans une seconde étape (b), on assemble à proportion égale l'ensemble des échantillons d'eaux chaudes récupérés pour chaque barrique du lot à contrôler de manière obtenir un mélange représentatif homogène. Dans le cas où le lot comporte un seul contenant, c'est l'échantillon récupéré du volume d'eau utilisé pour ce contenant qui servira pour la phase de dosage des contaminants.

Dans une étape (b'), on remplit un récipient 3 de 30 ml par exemple du mélange ainsi obtenu, ledit récipient étant fermé de manière étanche au moyen d'un bouchon inerte 2. Ce récipient est ensuite envoyé au laboratoire pour la phase de dosage des contaminants. Le récipient porte le même identifiant que le lot pour permettre une identification rapide. De préférence, ce récipient est à usage unique. Afin d'augmenter la stabilité du mélange avant de procéder à l'analyse, on incorpore avant son bouchage un agent stabilisant dans le mélange. Cet agent stabilisant peut être par exemple un composé du type de fluorure de sodium.

Les étapes (a), (b) et (b') sont réalisées sur site, c'est-à-dire directement par le fabricant des barriques lui-même ou par le viticulteur pour les barriques usagées.

La phase de dosage des contaminants comporte les étapes suivantes dans lesquelles :
- à l'étape (c), on détermine de manière expérimentale au préalable une valeur seuil de la concentration des contaminants ou le seuil de rejet au contrôle de contamination correspondant à un état de risque de contamination du contenant qui peut affecter la qualité du vin élevé dans un tel contenant contaminé. Cette valeur est déterminée à partir d'un test réalisé au préalable sur un même type de contenant. Généralement cette valeur seuil est inférieure au seuil de défaut perceptible. A titre d'exemple, le seuil de défaut organoleptique perceptible du TCA est de l'ordre de 1,5 à 3 ng/L, correspondant au seuil détectable par un dégustateur de vin.
- à l'étape (d), on réalise un dosage des contaminants du mélange contenu dans le récipient par diverses méthodes analytiques connues telles que la chromatographie en phase gazeuse associée à une spectrométrie de masse.
- à l'étape (e), on réalise une étude comparative entre la concentration mesurée par rapport à la valeur seuil pour déterminer si le lot est contaminé.

Lorsque la concentration est supérieure au seuil de rejet correspondant à un état contaminé du lot, il est alors nécessaire de procéder à une étape supplémentaire (f) qui consiste à répéter les opérations des étapes a) à e) pour chacun des contenants du lot contaminé individuellement de manière à localiser le ou les contenant(s) contaminé(s) du lot. La répétition des opérations est autorisée en vue de nouvelles analyses et ce au moins par trois fois sans risque de fausser les résultats des tests, en effet l'extraction des contaminants par les opérations d'échaudage est infime et partielle.

Ainsi cette étape permet d'identifier de manière très précise et quantitative le ou les contenants appartenant au même lot contaminé qui sont réellement contaminés et impropres à l'usage, et en particulier pour l'élevage d'un vin.

Il est à noter que le procédé de l'invention peut être mis en oeuvre pour contrôler la contamination de tout type de contenant en bois utilisé à l'élevage de toute boisson alcoolisée (vin, eau de vie, bière, etc...), il est particulièrement adapté pour déterminer les contaminants qui sont facilement extraits des bois du contenant tels que les composés du groupe des haloanisoles et les composés du groupes des halophénols dont la présence au de-là d'un certain seuil affecte le goût organoleptique de la boisson.

De manière générale les contaminants à doser peuvent être constitués par l'un des constituants du groupe des haloanisoles comprenant le 2,4,6-trichloroanisole (TCA), le 2,4,6-tribromoanisole (TBA), le 2,3,4,6-tétrachloroanisole (TeCA), et le pentachloroanisole (PCA), l'un des constituants du groupe des halophénols comprenant le 2,4,6-trichlorophénol (TCP), le 2,3,4,6 tétrachlorophénol (TeCP), et le pentachlorophénol (PCP), l'un des constituants de la famille des phénols volatils comprenant le 4-éthylphénol et le 4-éthylguaïacol, l'un des constituants du groupe d'hydrocarbure aromatique polycyclique, mais aussi l'acide acétique ou l'acétate d'éthyle.

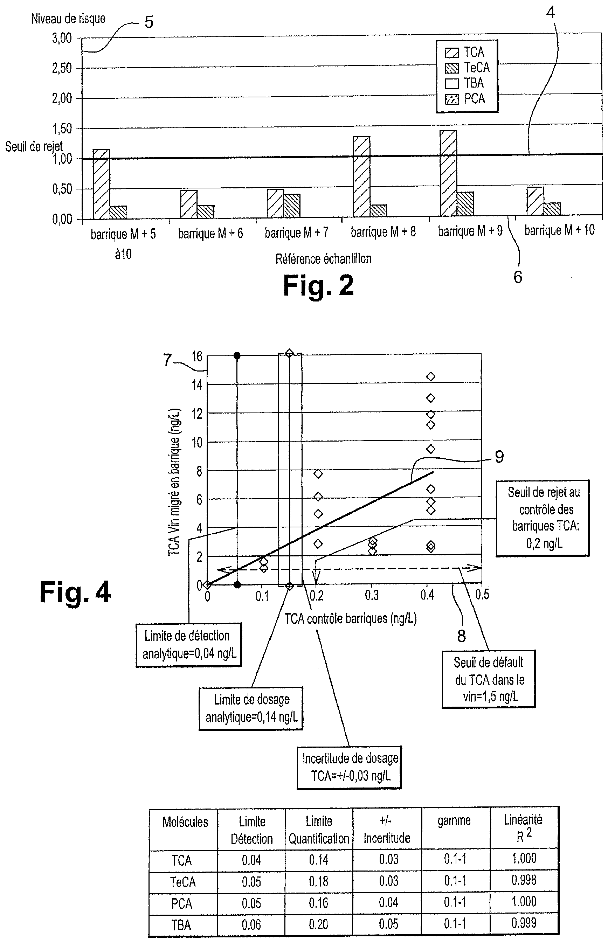

Une application du procédé tel que décrit ci-dessus au contrôle des contaminants TCA, TeCA, TBA et PCA qui font partis du groupe des haloanisoles va maintenant être décrite en se référant à la figure 2.

Le diagramme montré sur la figure 2 est un exemple de diagramme obtenu à l'issu de la phase de dosage, il représente le niveau de risque (axe Y référencé 5) mesuré pour chacun des 6 lots de barriques (axe X référencé 6).

Dans l'exemple illustré du procédé de l'invention, le seuil de rejet correspondant au trait horizontal référencé 4 est fixée ici à titre d'exemple à 1 qui correspond au rapport de la concentration en contaminant mesurée par le seuil d'acceptabilité de cette molécule. Au-delà de ce seuil, la concentration en contaminant du contenant est telle qu'il y a un risque de détérioration de la qualité organoleptique du vin élevé dans une telle barrique contaminée.

Bien évidemment, cette valeur peut être plus ou moins important en fonction du degré de risque de contamination.

Les résultats montrés concernent 6 lots de 5 barriques, chaque lot étant référencé par un identifiant, à savoir M+5, M+6, M+7, M+8, M+9 et M+10. Cet identifiant peut être par exemple un chiffre suivi du jour de prélèvement des eaux. Le mélange des eaux chaudes provenant de chaque barrique du lot est réalisé sur site par un opérateur. Cette phase de prélèvement et d'assemblage ne nécessite aucune compétence technique spécifique de la part de l'opérateur contrairement à la technique existante.

Les récipients sont envoyés pour l'analyse de préférence dans les 24 heures suivant le prélèvement afin d'éviter toute contamination ultérieure par le milieu environnant de stockage et de transport.

De préférence, les récipients sont hermétiquement fermés par un bouchon de matériau inerte.

Le diagramme montre que parmi les 6 lots analysés, les contaminants TBA et PCA ne sont pas détectés. Cette non détection ne correspond pas forcément à l'absence de ces contaminants, elle peut être due à une concentration trop faible pour être détectée par les méthodes analytiques utilisées. Par contre pour les lots portant les identifiants M+5, M+8 et M+9, le niveau de risque pour le TCA et TeCA est largement supérieur au seuil de rejet fixé. Il conviendra dans ce cas de passer à une étape supplémentaire (f) dans laquelle on répète les étapes a) à (e) pour contrôler la qualité de chaque barrique du lot M+5, M+8 et M+9 afin de déterminer individuellement la ou les barrique(s) contaminée(s).

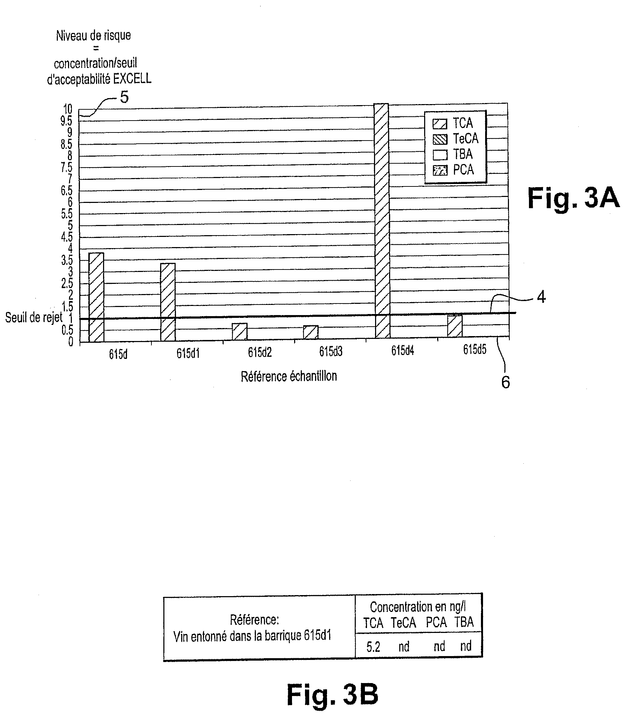

La figure 3.A et la figure 3.B illustrent un exemple de détermination individuelle de chaque barrique d'un lot contaminé référencé 615d constitué de 5 barriques. On a répété les étapes a) à e) pour chacune des barriques identifiées par une référence, à savoir 615d1, 615d2, 615d3, 615d4 et 615d5.

Le diagramme montré sur la figure 3.A dont l'axe Y représente le niveau de risque et l'axe X la référence échantillon montre que les barriques portants la référence 615d1 et 615d4 ne sont pas conforme au critère de qualité fixé, c'est-à-dire la concentration en TCA mesurée est supérieur au seuil de rejet fixé.

Pour démontrer la pertinence de ce contrôle de contamination, on a placé volontairement du vin rouge (12%) dans la barrique contaminée référencée 615d1 pendant 6 mois. On a analysé ensuite le vin au bout de cette période, en particulier le teneur en TCA, TeCA, PCA et TBA dans le vin pour connaître le taux de la quantité des molécules indésirables réellement migrées dans le vin rouge au cours de cette période. Le tableau de la figure 3.B montre clairement que le vin élevé dans une telle barrique est contaminé en TCA dans des teneur détectables à la dégustation, à savoir 5,2 ng/L, c'est-à-dire largement supérieure au seuil de perception organoleptique dans un vin rouge qui est généralement de l'ordre de 3 ng/L.

Ces tests montrent clairement qu'une détermination de la teneur en contaminants à partir des échantillons d'eaux chaudes permet d'exercer efficacement un contrôle de contamination des barriques. Ainsi grâce ce contrôle quantitatif de contamination de la ou les barrique(s), les fabricants de barriques peuvent soumettre ces barriques à un traitement approprié avant de les expédier aux viticulteurs.

La figure 4 montre des résultats qui permettent de faire correspondre la concentration des contaminants de la barrique déterminée à partir du procédé de l'invention par rapport à la concentration des contaminants réellement migrés dans le vin élevé dans un même type de barrique.

La courbe montré sur la figure 4 est une courbe de corrélation référencée 9, représentant la concentration de TCA de la barrique (axe X référencé 8) en fonction de la concentration de TCA migré dans le vin en barrique (axe Y référencé 7). La courbe obtenue est une droite ayant pour coefficient de linéarité autour de 1.

Le tableau de la figure 4 résume pour les autres composés de la même famille que le TCA.

Ainsi à partir de ces résultats, il est alors possible d'extraire le seuil de rejet au contrôle de barriques en limitant la courbe de corrélation par le seuil de défaut organoleptique perceptible représenté par un triait pointillé horizontal référencé 10, en tenant compte de l'incertitude de dosage, de la limite de détection analytique et de la limite de dosage analytique.

Dans l'exemple illustré sur la figure 4, pour le TCA, le seuil de défaut dans un produit fini tel que le vin du TCA est fixé à 1,5 ng/L, la limite de détection est fixée à 0,04 ng/L, la limite de dosage est fixée à 0,14 ng/L et l'incertitude de dosage est de +/- 0,03 ng/L, par conséquent le seuil de rejet au contrôle de barrique selon le procédé de la présente invention est de 0,2 ng/L, largement inférieure au seuil de défaut.

## Revendications

1. Procédé de contrôle de contamination d'un lot comprenant au moins un contenant en bois (1), destiné à l'élevage d'une boisson alcoolisée, **caractérisé en ce que** ledit procédé comporte :
une phase de préparation d'échantillon destiné au dosage des contaminants dans laquelle :
a) on récupère un échantillon d'un volume d'eau chaude ou à température ambiante qui a été mise en contact sous agitation avec toute la surface interne dudit au moins un contenant du lot à contrôler, ledit volume d'eau étant utilisé une seule fois pour chaque contenant;
b) ledit échantillon récupéré dans le cas où le lot comporte un seul contenant ou l'assemblage à proportion égale de l'ensemble des échantillons d'eaux récupérés pour chaque contenant du lot à contrôler est ensuite envoyé dans un récipient (3) fermé pour une phase de dosage des contaminants,
et une phase de dosage des contaminants dans laquelle :
c) on détermine au préalable une valeur seuil de la concentration des contaminants correspondant à un état de risque de contamination du contenant ;
d) on réalise un dosage des contaminants de l'échantillon récupéré ou de l'assemblage obtenu;
e) on réalise une étude comparative entre la concentration des contaminants mesurée par rapport à la valeur seuil pour déterminer si le lot est contaminé.

2. Procédé selon la revendication 1, **caractérisé en ce qu'**il comporte une étape supplémentaire f) dans laquelle lorsque la concentration est supérieure à la valeur seuil correspondant à un état contaminé du lot et lorsque ledit lot comprend au moins deux contenants, on répète les opérations des étapes a) à e) pour chacun des contenant dudit lot de manière à localiser le ou les contenant(s) contaminés du lot.

3. Procédé selon la revendication 1 ou 2, **caractérisé en ce que** dans l'étape b), ayant rempli le récipient (3) avec ledit échantillon récupéré ou avec ledit assemblage ainsi obtenu, ledit récipient étant fermé de manière étanche au moyen d'un bouchon inerte (2), on incorpore dans ledit récipient (3) avant son bouchage un agent stabilisant.

4. Procédé selon la revendication 3, **caractérisé en ce que** ledit agent stabilisant est un composé du type fluorure de sodium, métabisulfite de sodium, silicate de sodium, ou benzoate de sodium, la concentration de l'agent stabilisant étant inférieure à 100 mg/l.

5. Procédé selon l'une des revendications 1 à 4, **caractérisé en ce que** ledit lot est constitué d'au moins cinq contenants en bois.

6. Procédé selon l'une des revendications 1 à 5, **caractérisé en ce que** le dosage des contaminants est effectué dans les vingt-quatre heures suivant le prélèvement réalisé à l'étape b).

7. Procédé selon l'une des revendications 1 à 6, **caractérisé en ce que** le temps de la mise en contact de l'eau avec la surface interne du contenant est au moins supérieur à cent secondes.

8. Procédé selon l'une des revendications 1 à 7, **caractérisé en ce que** l'eau chaude utilisée dans l'étape a) est de l'eau portée à une température contrôlée comprise entre 50 et 80 °C.

9. Procédé selon la revendication 8, **caractérisé en ce que** l'on incorpore de l'éthanol dans l'eau de manière à ajuster la solvatation de l'eau.

10. Procédé selon la revendication 8 ou 9, **caractérisé en ce que** la qualité de l'eau utilisée lors de l'étape a) est contrôlée périodiquement, au moins une fois par mois.

11. Procédé selon l'une des revendications 8 à 10, **caractérisé en ce que** l'eau utilisée lors de l'étape a) est de l'eau utilisée pour contrôler l'étanchéité du contenant.

12. Procédé selon l'une des revendications 1 à 11, **caractérisé en ce que** les contaminants à doser sont constitués par l'un des constituants du groupe des haloanisoles comprenant les 2,4,6-trichloroanisoles (TCA), les 2,4,6-tribromoanisoles (TBA), les 2,3,4,6-tétrachloroanisoles (TeCA), et le pentachloroanisole (PCA), l'un des constituants du groupe des chlorophénols comprenant les 2,4,6-trichlorophénols (TCP), les 2,3,4,6 tétrachlorophénols (TeCP), et les pentachlorophénols (PCP), de la famille des phénols volatils comprenant le 4-éthylphénol et le 4-éthylguaïacol, l'un des constituants du groupe d'hydrocarbure aromatique polycyclique, l'acide acétique ou l'acétate d'éthyle.

13. Procédé selon l'une des revendications 1 à 12, **caractérisé en ce qu'**il comprend une étape supplémentaire dans laquelle pour chaque contaminant, on réalise une courbe de corrélation entre la concentration du contaminant dans la boisson après avoir passé un temps déterminé dans le contenant et la concentration du contaminant correspondant du contenant, de manière à extraire la valeur seuil de la concentration des contaminants.

14. Procédé selon la revendication 13, **caractérisé en ce que** la valeur seuil de rejet au contrôle de contamination pour le contaminant TCA est comprise entre 0,2ng/L et 1ng/L.

15. Procédé selon l'une des revendications 1 à 14, **caractérisé en ce que** les contenants sont des barriques de bois, notamment en chêne, neuves ou usagées destinées à l'élevage des vins ou des spiritueux en général.

## Patentansprüche

1. Verfahren zur Kontaminationskontrolle einer Charge, die mindestens einen Holzbehälter (1) umfasst, der zum Ausbau eines alkoholischen Getränks vorgesehen ist, **dadurch gekennzeichnet, dass** das Verfahren umfasst:
eine Phase zur Vorbereitung einer Probe, die zur Dosierung der Kontaminanten vorgesehen ist, in der:
a) eine Probe eines Volumens von heißem oder auf Umgebungstemperatur befindlichem Wasser gewonnen wird, das unter Rühren mit der gesamten Innenfläche des mindestens einen Behälters der zu kontrollierenden Charge in Kontakt gebracht wurde, wobei das Wasservolumen ein einziges Mal für jeden Behälter verwendet wird;
b) die gewonnene Probe in dem Fall, in dem die Charge einen einzigen Behälter umfasst, oder der Verschnitt zu gleichen Teilen der Gesamtheit der Wasserproben, die für jeden Behälter der zu kontrollierenden Charge gewonnenen wurden, anschließend in einen geschlossenen Behälter (3) geleitet wird für eine Phase zur Dosierung der Kontaminanten,
und eine Phase zur Dosierung der Kontaminanten, in der:
c) vorab ein Schwellenwert der Konzentration der Kontaminanten bestimmt wird, der einem Zustand von Kontaminationsgefahr des Behälters entspricht;
d) eine Dosierung der Kontaminanten der gewonnenen Probe oder des gewonnenen Verschnitts ausgeführt wird;
e) eine vergleichende Untersuchung zwischen der Konzentration der gemessenen Kontaminanten in Bezug auf den Schwellenwert ausgeführt wird, um zu bestimmen, ob die Charge kontaminiert ist.

2. Verfahren nach Anspruch 1, **dadurch gekennzeichnet, dass** es einen zusätzlichen Schritt f) umfasst, in dem, wenn die Konzentration mehr als den Schwellenwert beträgt, der einem kontaminierten Zustand der Charge entspricht, und wenn die Charge mindestens zwei Behälter umfasst, die Vorgänge der Schritte a) bis e) für jeden der Behälter der Charge wiederholt werden, um den oder die kontaminierten Behälter der Charge zu lokalisieren.

3. Verfahren nach Anspruch 1 oder 2, **dadurch gekennzeichnet, dass** im Schritt b), nachdem der Behälter (3) mit der gewonnenen Probe oder mit dem so gewonnenen Verschnitt befüllt wurde, wobei der Behälter in dichter Weise mittels eines inerten Verschlusses (2) verschlossen wird, vor dessen Verschließen ein Stabilisierungsmittel in den Behälter (3) eingebracht wird.

4. Verfahren nach Anspruch 3, **dadurch gekennzeichnet, dass** das Stabilisierungsmittel eine Verbindung vom Typ Natriumfluorid, Natriummetabisulfit, Natriumsilikat oder Natriumbenzoat ist, wobei die Konzentration des Stabilisierungsmittels weniger als 100 mg/l beträgt.

5. Verfahren nach einem der Ansprüche 1 bis 4, **dadurch gekennzeichnet, dass** die Charge aus mindestens fünf Holzbehältern besteht.

6. Verfahren nach einem der Ansprüche 1 bis 5, **dadurch gekennzeichnet, dass** die Dosierung der Kontaminanten in den vierundzwanzig Stunden erfolgt, die der im Schritt b) ausgeführten Entnahme folgen.

7. Verfahren nach einem der Ansprüche 1 bis 6, **dadurch gekennzeichnet, dass** die Zeit des Inkontaktbringens des Wassers mit der Innenfläche des Behälters mindestens mehr als einhundert Sekunden beträgt.

8. Verfahren nach einem der Ansprüche 1 bis 7, **dadurch gekennzeichnet, dass** das im Schritt a) verwendete heiße Wasser Wasser ist, das auf eine kontrollierte Temperatur im Bereich zwischen 50 und 80 °C gebracht wird.

9. Verfahren nach Anspruch 8, **dadurch gekennzeichnet, dass** Ethanol in das Wasser eingebracht wird, um die Solvatation des Wassers einzustellen.

10. Verfahren nach Anspruch 8 oder 9, **dadurch gekennzeichnet, dass** die Qualität des beim Schritt a) verwendeten Wassers regelmäßig, mindestens einmal pro Monat, kontrolliert wird.

11. Verfahren nach einem der Ansprüche 8 bis 10, **dadurch gekennzeichnet, dass** das beim Schritt a) verwendete Wasser Wasser ist, das verwendet wird, um die Dichtigkeit des Behälters zu kontrollieren.

12. Verfahren nach einem der Ansprüche 1 bis 11, **dadurch gekennzeichnet, dass** die zu dosierenden Kontaminanten bestehen aus einem der Bestandteile der Gruppe der Haloanisole, die die 2,4,6-Trichloranisole (TCA), die 2,4,6-Tribromanisole (TBA), die 2,3,4,6-Tetrachloranisole (TeCA) und Pentachloranisol (PCA) umfasst, einem der Bestandteile der Gruppe der Chlorphenole, die die 2,4,6-Trichlorphenole (TCP), die 2,3,4,6-Tetrachlorphenole (TeCP), und die Pentachlorphenole (PCP) umfasst, der Familie der flüchtigen Phenole, die 4-Ethylphenol und 4-Ethylguajacol umfasst, einem der Bestandteile der Gruppe aus polycyclischem aromatischem Kohlenwasserstoff, Essigsäure oder Ethylacetat.

13. Verfahren nach einem der Ansprüche 1 bis 12, **dadurch gekennzeichnet, dass** es einen zusätzlichen Schritt umfasst, in dem, für jeden Kontaminanten eine Korrelationskurve zwischen der Konzentration des Kontaminanten im Getränk, nachdem es eine bestimmte Zeit im Behälter verbracht hat, und der Konzentration des entsprechenden Kontaminanten des Behälters ausgeführt wird, um den Schwellenwert der Konzentration der Kontaminanten zu extrahieren.

14. Verfahren nach Anspruch 13, **dadurch gekennzeichnet, dass** der Schwellenwert zur Ablehnung in der Kontaminationskontrolle für den Kontaminanten TCA im Bereich zwischen 0,2 ng/L und 1 ng/L beträgt.

15. Verfahren nach einem der Ansprüche 1 bis 14, **dadurch gekennzeichnet, dass** die Behälter Holzfässer sind, insbesondere aus Eiche, neu oder gebraucht, die zum Ausbau der Weine oder der Spirituosen im Allgemeinen vorgesehen sind.

## Claims

1. Method of monitoring the contamination of a batch comprising at least one wooden container (1), intended for maturing an alcoholic beverage, **characterised in that** said method comprises:
a phase of preparing a sample intended for assaying contaminants in which:
a) a sample of a volume of hot water or water at ambient temperature is recovered after having been brought into contact, with stirring, with the entire internal surface of said at least one container of said batch to be monitored, said volume of water being used only once for each container;
b) said sample recovered in the case where the batch comprises a single container where the assembly in equal proportions of all of the samples of water recovered for each container of said batch to be monitored is then sent to a closed vessel (3) for a phase of assaying contaminants, and a phase of assaying contaminants wherein:
c) a threshold value of the concentration of contaminants corresponding to a state of risk of contamination of the container is determined first;
d) an assay of the contaminants of the sample recovered or of the assembly obtained is carried out;
e) a comparative study is carried out between the concentration of contaminants measured relative to the threshold value in order to determine whether the batch is contaminated.

2. Method according to claim 1, **characterised in that** it comprises an additional step f) wherein when the concentration is greater than the threshold value corresponding to a contaminated state of the batch and when said lot comprises at least two containers, the operations of the steps a) to e) are repeated for each one of the containers of said batch in such a way as to locate the contaminated container or containers of the batch.

3. Method according to claim 1 or 2, **characterised in that** in the step b), having filled the vessel (3) with said recovered sample or with said assembly obtained as such, said vessel being sealed in a leak tight manner by means of an inert stopper (2), a stabiliser is incorporated into said vessel (3) before the stopping thereof.

4. Method according to claim 3, **characterised in that** said stabiliser is a compound of the sodium fluoride, sodium metabisulfite, sodium silicate, or sodium benzoate type, the concentration of the stabiliser being less than 100 mg/l.

5. Method according to one of claims 1 to 4, **characterised in that** said batch is comprised of at least five wooden containers.

6. Method according to one of claims 1 to 5, **characterised in that** the assaying of contaminants is carried out within twenty-four hours following the sampling carried out in the step b) .

7. Method according to one of claims 1 to 6, **characterised in that** the contact time of the water with the internal surface of the container is at least greater than one hundred seconds.

8. Method according to one of claims 1 to 7, **characterised in that** the hot water used in the step a) is water brought to a controlled temperature between 50 and 80°C.

9. Method according to claim 8, **characterised in that** ethanol is incorporated into the water in such a way as to adjust the solvation of the water.

10. Method according to claim 8 to 9, **characterised in that** the water used during the step a) is periodically controlled, at least once a month.

11. Method according to claim 8 to 10, **characterised in that** the water used in the step a) is water used to control the seal of the container.

12. Method according to claim 1 to 11, **characterised in that** the contaminants to be assayed are comprised by one of the constituents of the group of haloanisoles comprising 2,4,6-trichloroanisoles (TCA), 2,4,6-tribromoanisoles (TBA), 2,3,4,6-tetrachlorianisoles (TeCA), and pentachloroanisole (PCA), one of the constituents of the group of chlorophenols comprising 2,4,6-trichlorophenols (TCP), 2,3,4,6 tetrachlorophenols (TeCP), and pentachlorophenols (PCP), of the family of volatile phenols comprising 4-ethylphenol and 4-ethylguaiacol, one of the constituents of the group of polycyclic aromatic hydrocarbons, acetic acid or ethyl acetate.

13. Method according to one of claims 1 to 12, **characterised in that** it comprises an additional step wherein for each contaminant, a correlation curve is carried out between the concentration of the contaminant in the beverage after having spent a determined time in the container and the concentration of the corresponding contaminant of the container, so as to extract the threshold value of the concentration of the contaminants.

14. Method according to claim 13, **characterised in that** the reject threshold value at the contamination control for the TCA contaminant is between 0.2 ng/L and 1 ng/L.

15. Method according to one of claims 1 to 14, **characterised in that** the containers are wooden barrels, in particular made of oak, new or used intended for the maturing of wines or spirits in general.
